# EUROPEAN PATENT SPECIFICATION

(11) **EP 2 520 660 B1**
(45) Date of publication and mention of the grant of the patent: **24.01.2018**
(21) Application number: 10840314.8
(22) Date of filing: 29.12.2010
(51) Int. Cl.: C12Q 1/68

(54) **KITS FOR QUANTITATIVE DETECTION OF K-RAS MUTATIONS**
KITS FÜR DEN QUANTITATIVEN NACHWEIS VON K-RAS-MUTATIONEN
KITS POUR LA DÉTECTION QUANTITATIVE DES MUTATIONS DE K-RAS

(30) Priority: 30.12.2009 CN 200910215835
(43) Date of publication of application: 07.11.2012
(73) Proprietor: Beijing ACCB Biotech Ltd., Beijing 100094 (CN)
(72) Inventor: XU, Junpu, Beijing 100094 (CN); CHEN, Zhao, Beijing 100094 (CN)
(74) Representative: Dossmann, Gérard
(86) International application number: PCT/CN2010/002201
(87) International publication number: WO 2011/079524

(56) References cited:
- EP-A1- 1 829 964
- EP-A1- 1 905 842
- WO-A1-2010/071821
- WO-A2-99/49293
- WO-A2-2008/112269
- CN-A- 101 423 866
- CN-A- 101 434 985
- CN-A- 101 608 241
- A. LIEVRE ET AL: "KRAS Mutations As an Independent Prognostic Factor in Patients With Advanced Colorectal Cancer Treated With Cetuximab", JOURNAL OF CLINICAL ONCOLOGY, vol. 26, no. 3, 20 January 2008 (2008-01-20), pages 374-379, XP055036677, ISSN: 0732-183X, DOI: 10.1200/JCO.2007.12.5906
- DxS Limited: "TheraScreen®: K-RAS Mutation Kit", , February 2009 (2009-02), XP002698331, Retrieved from the Internet: URL:http://www.google.nl/url?sa=t&rct=j&q= &esrc=s&source=web&cd=5&ved=0CE0QFjAE&url= http%3A%2F%2Fwww.oem-info.com%2Froche%2Fha ndbooks%2Fdata%2FDU001g_KRAS_TheraScreen_E NGLISH.pdf&ei=1nmwUf_XHaGw4QTUiIGIAw&usg=A FQjCNEzD-q5udK9X3GPNA0C8eDycvC-tQ&bvm=bv.4 7534661,d.ZWU [retrieved on 2013-06-06]

## Description

### BACKGROUND OF THE INVENTION

K-ras belongs to ras oncogene family, it is also referred as p21 gene because it encodes 21kD ras protein. K-ras transduces an important signal cascade. The encoded product of K-ras gene locates in the downstream of K-ras signaling cascade, functioning as an important "switch" during tumor cell growth, proliferation and angiogenesis. When mutation occurs in K-ras gene, it keeps K-ras in a constitutively active state, leading to deregulation of cell growth and inhibition of apoptosis. The mutation rate of K-ras in different tumor tissues are various, wherein pancreas cancer is 82%, colon cancer is 43%, lung cancer is 30%, hypothyroid cancer is 29%, bladder, liver, kidney and cervical cancer are 10% or lower (Bos J.L. et al, Cancer Res, 1989, 49(17):4682-4689). Because the constitutive activation of mutant K-ras, patients have poor response to anti-EGFR therapy which targets to inhibit signaling upstream of K-ras (Amado R.G. et al, J. Clin. Oncol., 2008, 26(10): 1626-1634; Lièvre A. et al, J. Clin. Oncol., 2008; 26(3):374-379). Therefore, by detecting the mutation(s) of K-ras gene, it is possible to predict the therapeutic efficacy of anti-EGFR, in order to realize individualized therapy for cancer patients and predict prognosis.

At present, the mutation sites of K-ras gene are mainly gathered at Codon 12 and 13 (Zhang Jianjie et al., Journal of Xi'an Jiaotong University, 2005, 26(4):349-351; He Xiaowen et al., Chinese Journal of Digestion, 2002, 22(1): 26-28), wherein the most common mutation is Codon 12 GGT→GTT or GGT → GAT. By detecting five type of mutations in K-ras (a tumor-related gene) Codon 12 and 13, which are related to the effect of targeted molecular anti-cancer therapeutics, the present invention can predict the therapeutic effect of the targeted molecular drugs such as Erbitux, by using real-time quantitative PCR.

The detecting method of the present invention has the following advantages: easy manipulation, and easy standardization. Other methods, such as allele specific oligonucleotide probe hybridization method, are very much dependent on hybridization conditions, and therefore require strict control of the experimental conditions. The restriction fragment length polymorphism method, on the other hand, needs a lot of human labor, and can not generate quantitative results. The method of the present invention has short experimental cycle, and can be completed with 2 hours. It doesn't need verification the results by sequencing, whereas the direct sequencing and high resolution melting analysis need 4 days to 2 weeks. Sensitivity of the method of the present invention is high, which, after optimizing experimental conditions, can reach 1% for detecting mutations, whereas sensitivity of direct sequencing is 20-50%. Specificity of the method of the present invention is also high. Immunohistochemistry (IHC) method can easily get pseudo-positive and pseudo-negative results, and can not determine the position and types of point mutations. The unique advantage of the present invention is accurate quantification. By using absolute quantification method to analyze data, draw standard curve, and accurately determine the content of wild-type gene and mutant gene in the samples, one can obtain ratio of the mutant gene in the samples, which will be helpful for clinical diagnosis and therapeutic selection. Furthermore, the present invention is safe and non-toxic, other methods such as chemical breaking method of mismatch base need isotope and toxic chemical agents.

### SUMMARY OF THE INVENTION

The question that the present invention addresses is to provide an assay kit for quantitatively detecting an K-ras gene mutation, which can quantitatively detect the following mutations: GGT at position 2155 in K-ras Codon 12 (SEQ ID NO:1; SEQ ID NO:2) replaced with GTT, AGT, GAT or TGT; or GGC in Codon 13 (SEQ ID NO:1; SEQ ID NO:3) replaced with GAC.

To address the above question, the present invention provides quantitative detection kit containing a mixture comprising Taq enzyme, 10 x Taq buffer, MgCl₂, dNTP mixture, PCR primers which can specifically amplify the sequences at K-ras gene mutation positions, and probes which can specifically identify wild-type sequences and mutant sequences, together with method of the detection, as follows:
(1) Separately design upstream and downstream primers around the mutation positions of Codon 12 and 13 of K-ras gene; and design specific probes according to each mutant site. Said probes can specifically bind wild-type sequences or the mutant sequences to be detected at specific K-ras sites, so as to determine whether the tested mutations occur at said sites.
(2) To accurately and quantitatively determine the ratio of the K-ras mutations, standards were designed in the present invention.
(3) Use fluorescent quantitative PCR to detect the samples and standards.
(4) Obtain standard curves for quantitative detection from the detection results of the standards, and calculate the ratios of K-ras gene mutations to the total wild type K-ras gene in the samples to be tested.

Prior to said step (1) it further includes: extracting nucleic acid from the samples, purifying it and determining the content of it.

The probes for fluorescent quantitative PCR specifically bind the sequences at K-ras gene mutation sites under suitable PCR conditions. Preferably, said probes link a fluorescence emitting group at their 5' end, and link a fluorescence quenching group at their 3' end. Said fluorescence emitting group is selected from FAM, TET, HEX and ROX. Said fluorescence quenching group is selected from BHQ, TAMARA. Preferably, said emitting group is FAM, and said quencher group is BHQ. Preferably, the sequences of said probes are selected from the group consisting of SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, and SEQ ID NO: 28.

Said standards include at least one of plasmids, genome DNA or chemically synthesized sequences. Preferably, said standards comprises a wild-type plasmid, a mutant plasmid, or both a wild-type plasmid and a mutant plasmid, wherein said wild-type plasmids include wild-type sequences of K-ras gene, and said mutant plasmids include mutant sequences of K-ras gene. More preferably, said standards are consisted of a wild-type plasmid, a mutant plasmid, or both a wild-type plasmid and a mutant plasmid.

The tested samples include fresh tissue, paraffin embedded tissues, cell lines, blood, pleural effusion, peritoneal effusion, saliva, digestive juice, urine and feces.

Said primers consist of upstream primers and downstream primers. Preferably, said primers are selected from the group consisting of SEQ ID NO: 4, SEQ ID NO: 5, SEQ ID NO: 6, and SEQ ID NO: 7.

Said quantitative detection kit for K-ras gene mutations includes the agents selected from: the above-mentioned primers, probes and standards. Preferably, said kit further includes Taq enzyme, 10 x Taq buffer, MgCl₂, and dNTP mixture. Preferably, the ratio of said primer to probe is 2:1-10:1, and said primer comprises a forward primer and reverse primer in a ratio of 1:3-3:1. Said standards include a mixture of said plasmids in a certain ratio, wherein the ratio of the content of wild-type plasmids to mutant plasmids is 0%-100%.

### BRIEF DESCRIPTION OF DRAWINGS

The accompanying drawings, which are incorporated in and form a part of this specification, illustrate embodiments of the technology and together with the description.

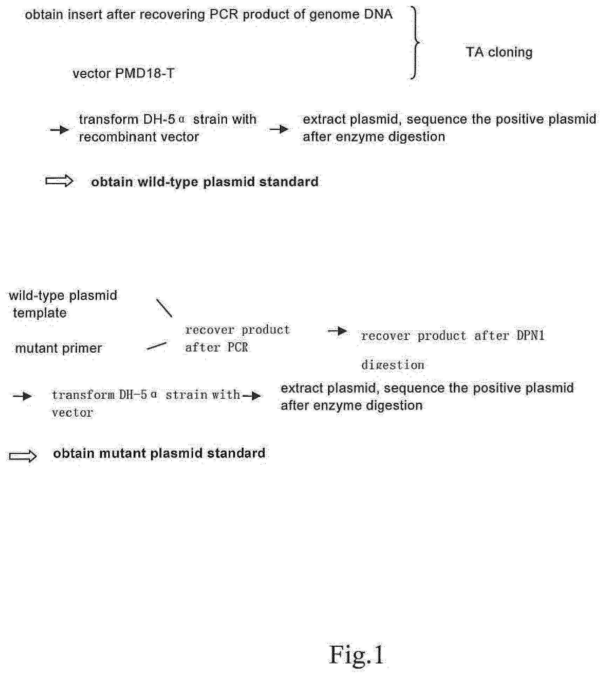

Figure 1 is a diagram showing the method for constructing the plasmid standards in Example 2.

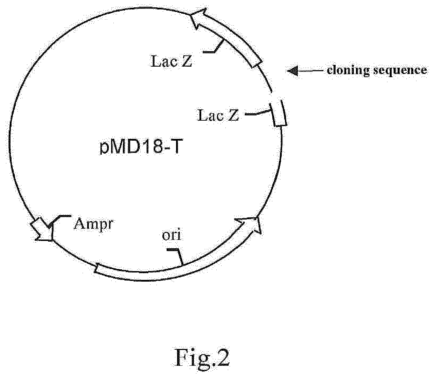

Figure 2 is a diagram showing the wild-type plasmid profile of Example 2, wherein the wild-type PCR product sequence is inserted into the carrier at the position marked with an arrow.

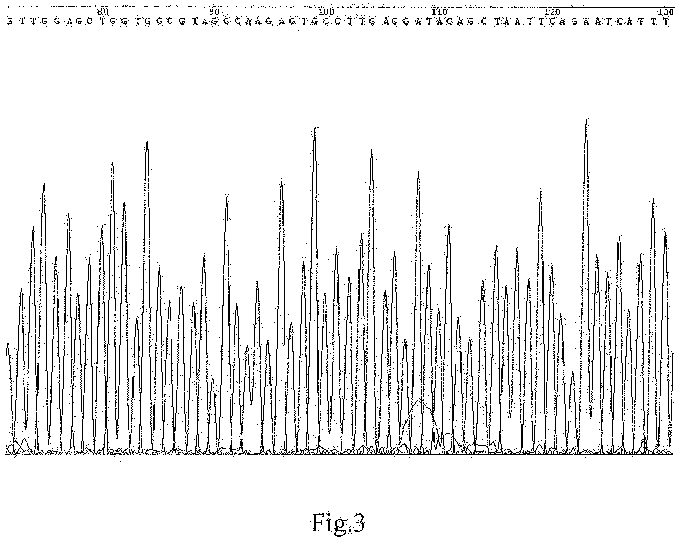

Figure 3 is a diagram showing the result of sequencing the wild-type plasmid standard of Example 2,

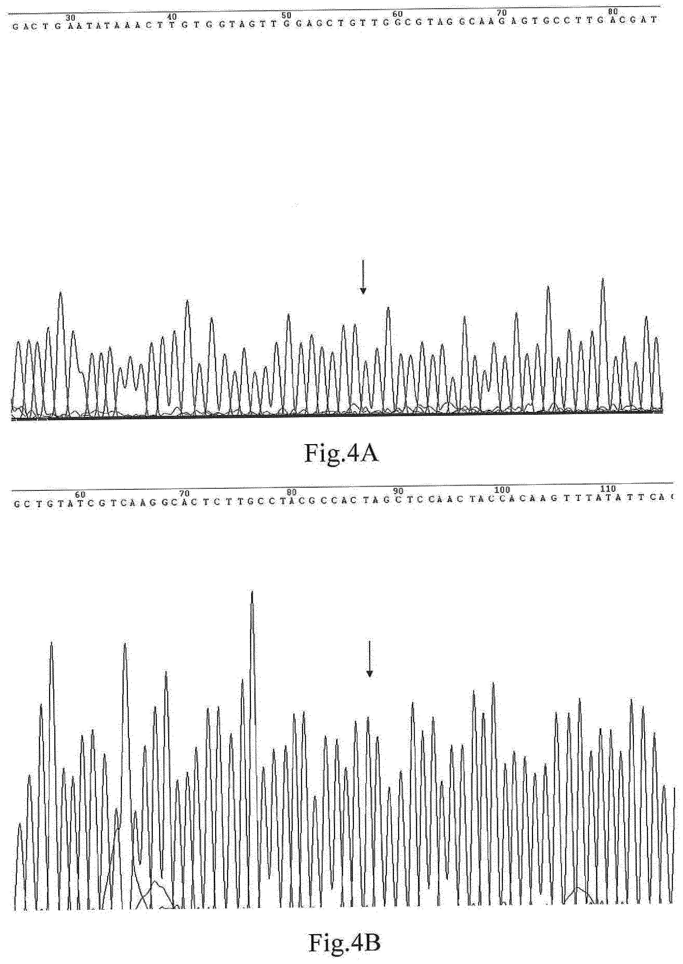

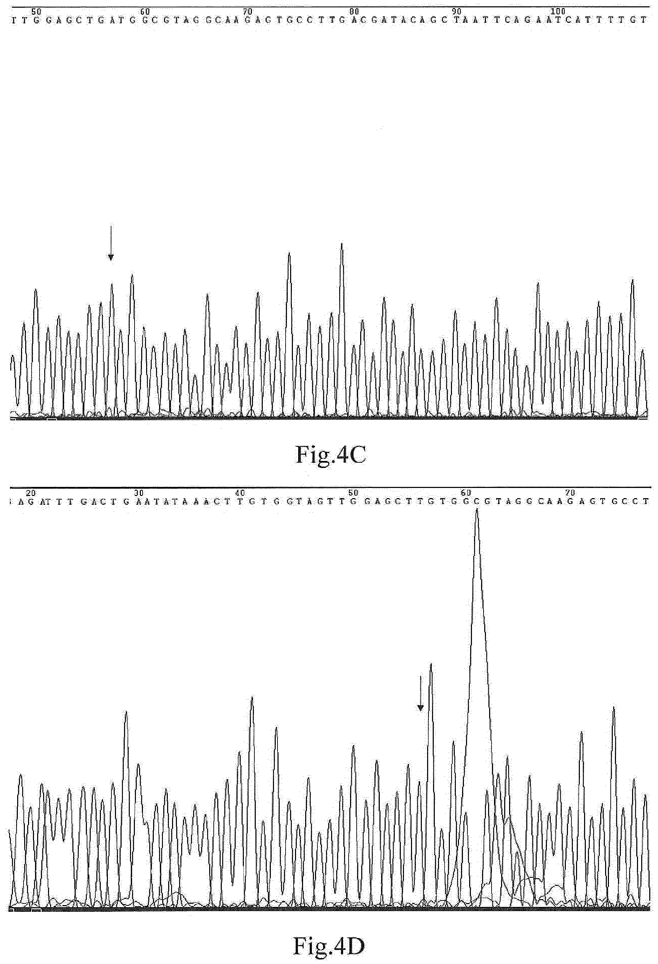

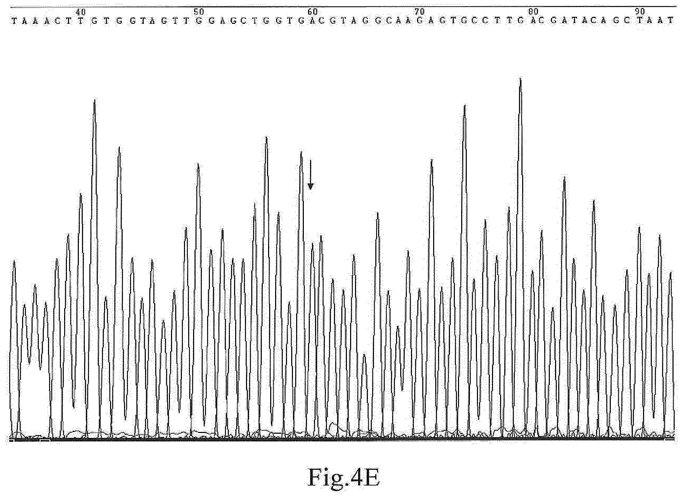

Figure 4 is a diagram showing the result of sequencing the mutant plasmid standard of Example 2, wherein Fig.A is the sequencing result of K-ras Codon 12 GGT→GTT mutant plasmid, Fig.B is the sequencing result of K-ras Codon 12 GGT→AGT mutant plasmid, Fig.C is the sequencing result of K-ras Codon 12 GGT→GAT mutant plasmid, Fig.D is the sequencing result of K-ras Codon 12 GGT → TGT mutant plasmid, Fig.E is the sequencing result of K-ras Codon 13 GGC → GAC mutant plasmid.

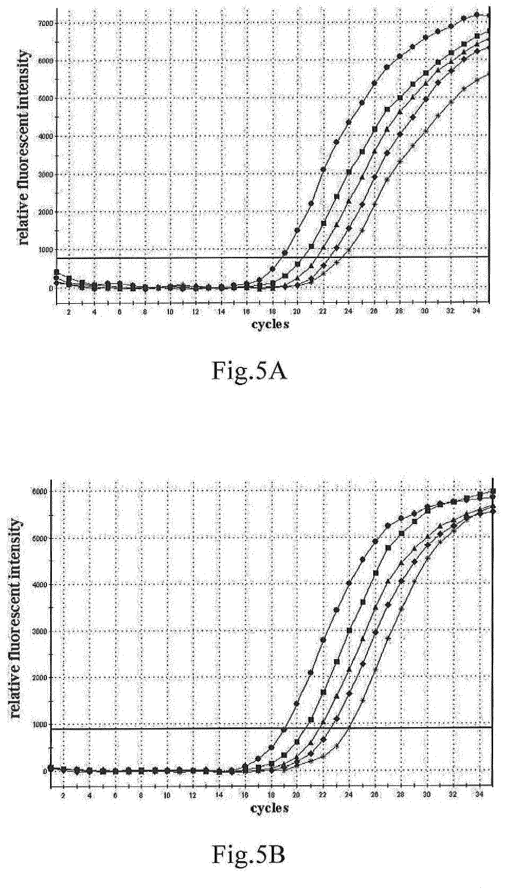

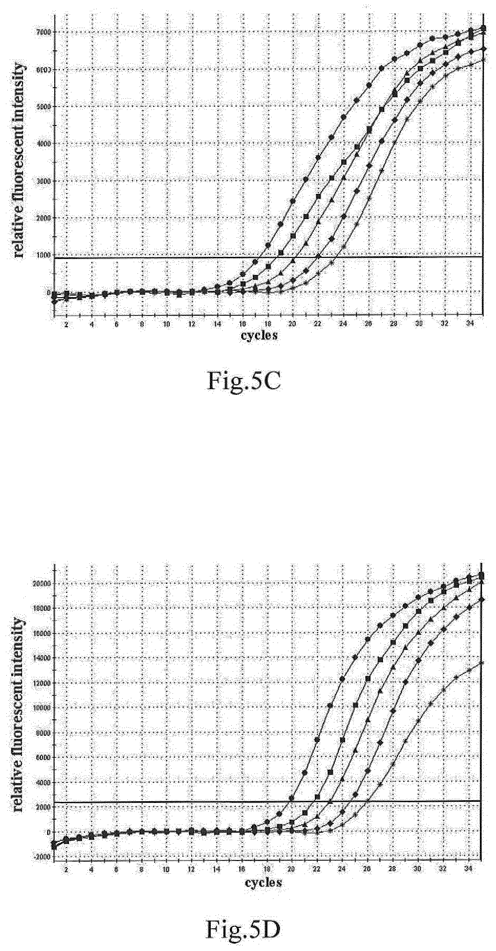

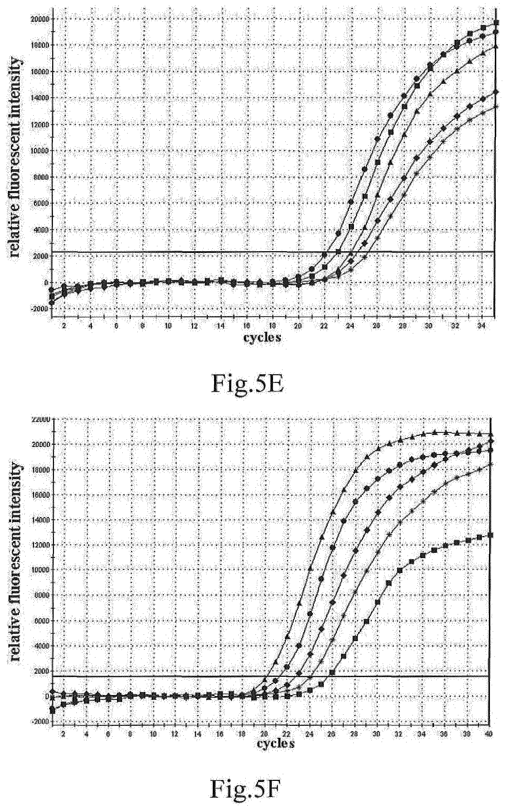

Figure 5 is a diagram showing the amplification curve of the standard of Example 3. Fig.A shows the amplification curve of K-ras wild-type plasmid standard; Fig.B shows the amplification curve of K-ras Codon 12 GGT → GTT mutant plasmid standard; Fig.C shows the amplification curve of K-ras Codon 12 GGT → AGT mutant plasmid standard; Fig.D shows the amplification curve of K-ras Codon 12 GGT → GAT mutant plasmid standard; Fig.E shows the amplification curve of K-ras Codon 12 GGT → TGT mutant plasmid standard; and Fig.F shows the amplification curve of K-ras Codon 13 GGC→GAC mutant plasmid standard.

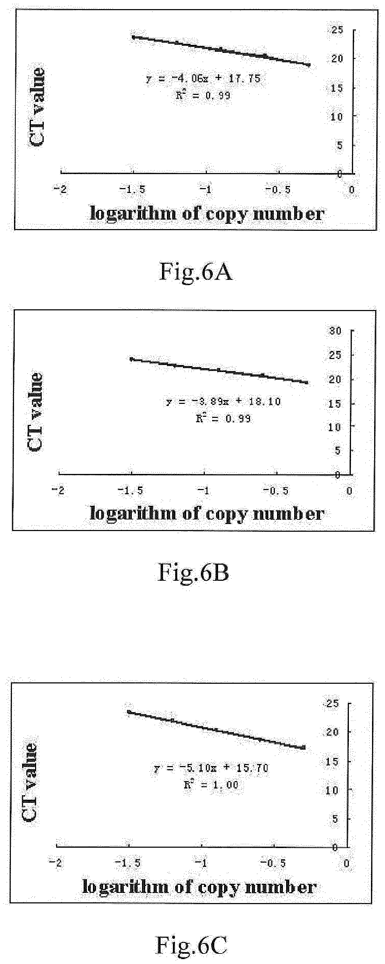

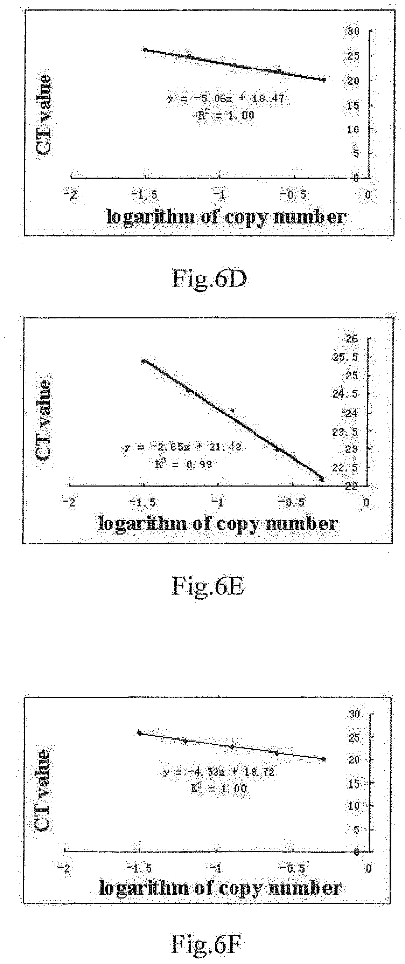

Figure 6 shows standard curves based on Figure 4. Fig.A shows the standard curve of K-ras wild-type plasmid; Fig.B shows the standard curve of K-ras Codon 12 GGT→GTT mutant plasmid; Fig.C shows the standard curve of K-ras Codon 12 GGT→AGT mutant plasmid; Fig.D shows the standard curve of K-ras Codon 12 GGT→GAT mutant plasmid; Fig.E shows the standard curve of K-ras Codon 12 GGT→TGT mutant plasmid; and Fig.F shows the standard curve of K-ras Codon 13 GGC→GAC mutant plasmid.

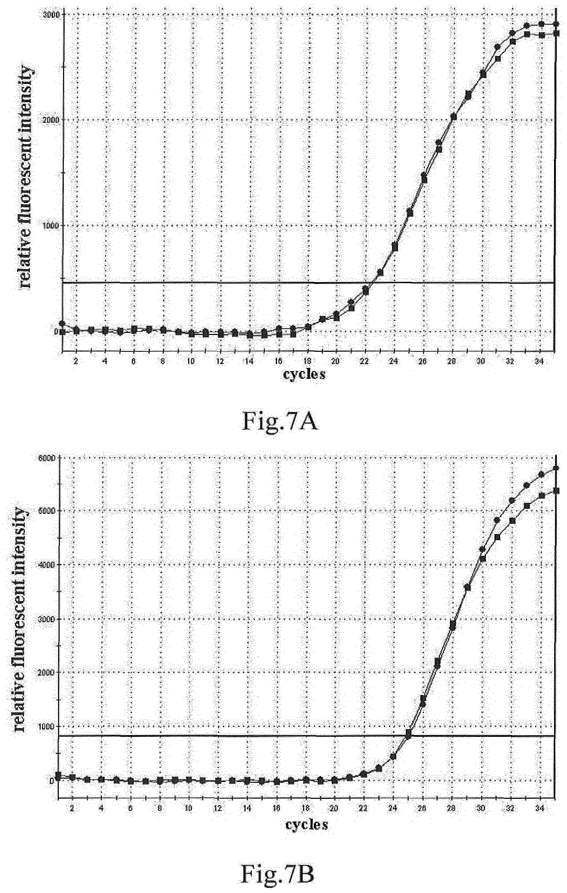

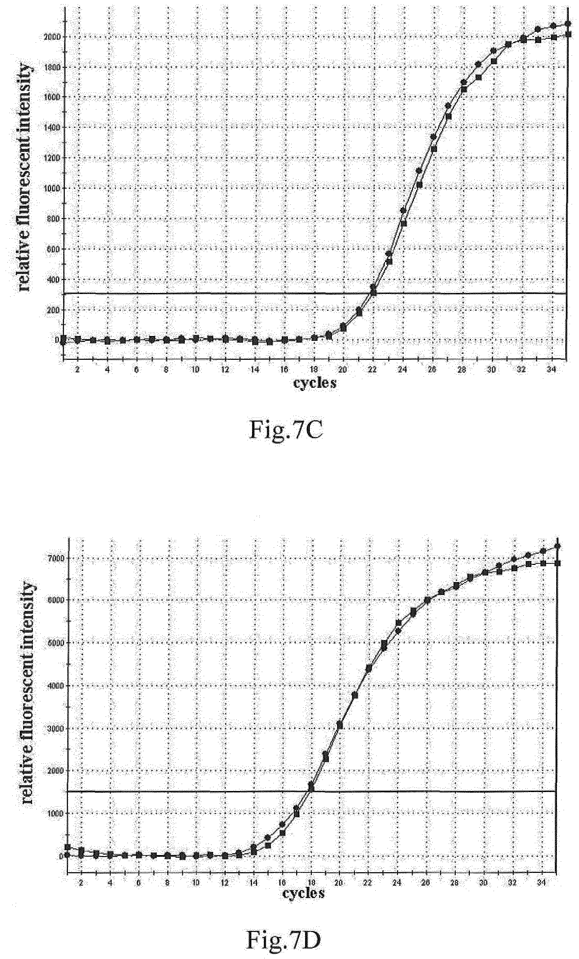

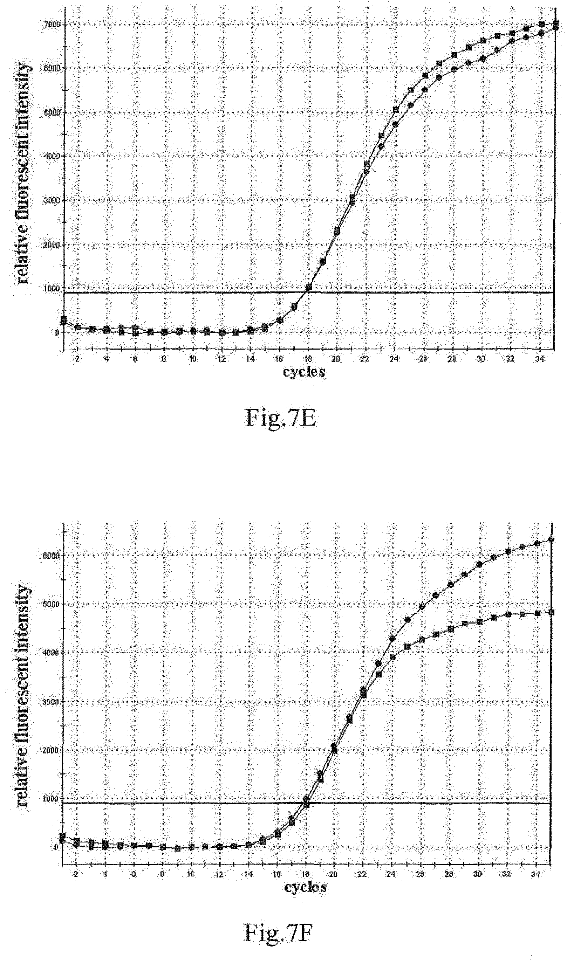

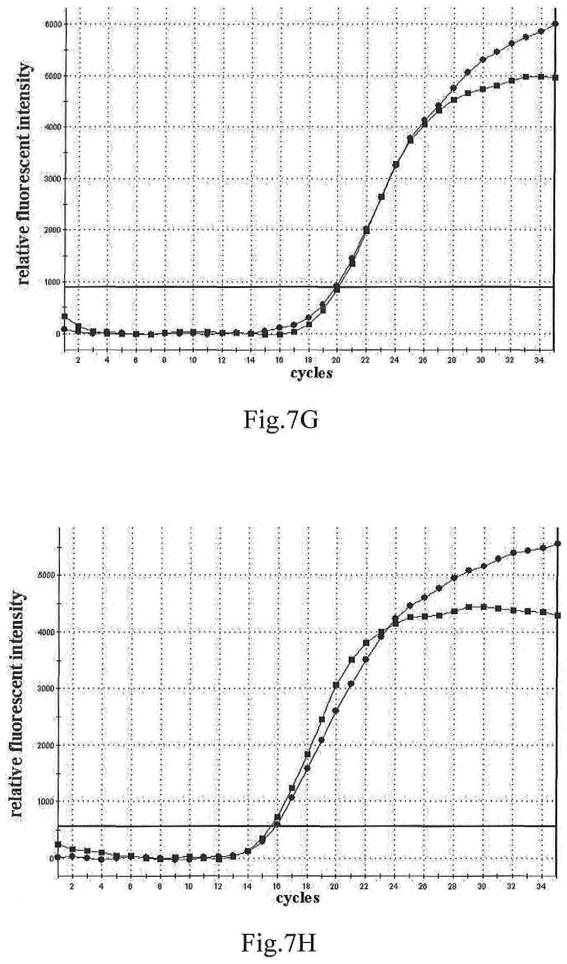

Figure 7 shows the amplification curve of fluorescent quantitative PCR of the wild-type (Fig.A) and GGT→TGT mutant (Fig.B) of K-ras Codon 12 in a paraffin embedded tissue sample; wild-type (Fig.C) and GGT→GTT mutant (Fig.D) of K-ras Codon 12 in a fresh tissue sample; wild-type (Fig.E) and GGC→GAC mutant (Fig.F) of K-ras Codon 13 in whole blood sample; and wild-type (Fig.G) and GGT→AGT mutant (Fig.H) of K-ras Codon 12 in cell line sample.

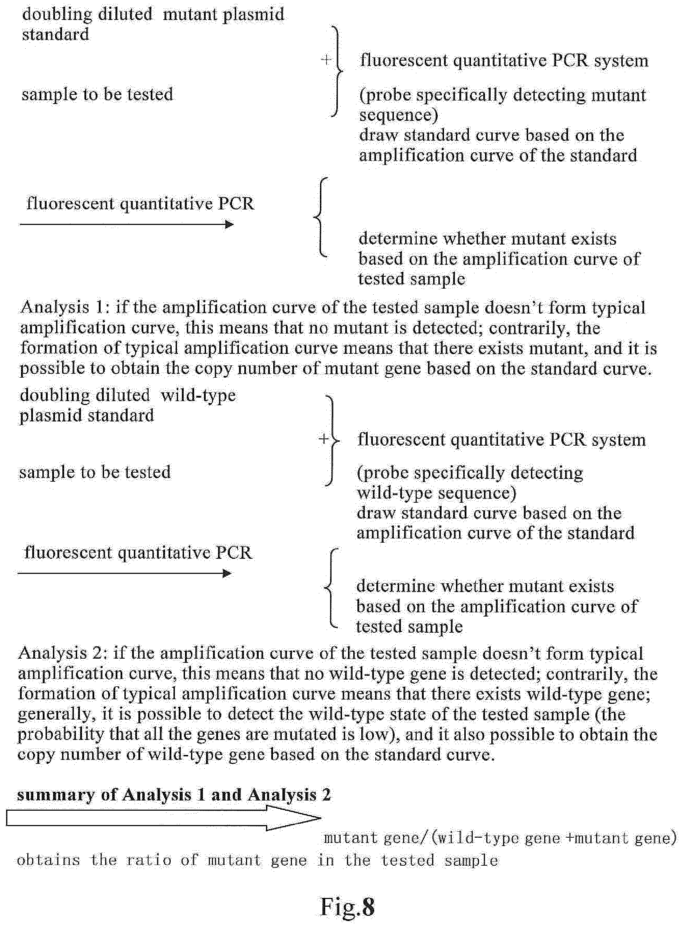

Figure 8 is a diagram of the quantitative method of the present invention.

### DETAILED DESCRIPTION OF PARTICULAR EMBODIMENTS

### Examples

The following examples are put forth so as to provide those of ordinary skill in the art with a complete disclosure and description of how to make use of the present invention, and are neither intended to limit the scope of what the inventors regard as their invention nor are they intended to represent that the experiments below are all or the only experiments performed. The experimental conditions not indicated in the Examples, are generally conventional, such as those disclosed in "Molecular Cloning, A Laboratory Manual, 3rd ed, (Sambrook J.)", or those suggested by the manufacturer.

### Example 1: Extracting genome DNA from fresh human tumor tissues, paraffin embedded tissues, peripheral blood, pleural effusion, and human cell lines

The tumor cell lines we tested included cell lines of: non-small-cell carcinoma (NSCLC; A549, H460, H838 and H1703), breast cancer (MCF-7, BT474 and HuL100), malignant mesothelioma (H513, H2052, H290, MS-1 and H28), colon cancer (SW480), head and neck cancer (U87), cervical carcinoma (Hela), sarcoma (Mes-SA, Saos-2 and A204).

The fresh human tumor tissues, peripheral blood, paraffin embedded tissues we tested included: NSCLC, mesothelioma, colon cancer, malignant melanoma, renal carcinoma, esophagus cancer, thyroid carcinoma, malignant cancer and ovarian cancer.

### Extraction of sample DNA

DNA extracting kit from Qiagen Inc., Promega Inc., or Roche Inc. can be used to extract genomic DNA from the samples. Content and purity of the extracted DNA can be determined by using Nanodrop ND1000 (Gene Inc.) (OD260/OD280 is about 1.8, OD260/OD230 is more than 2.0). For example, the sample DNA may be extracted using the DNA Extracting Kit (Promega Inc.) as follows:

### 1. DNA extraction from fresh tissues

(1) cut a bean-sized tissue using scissors, put it into a mortar, cut it into pieces, and ground it into powder by adding liquor nitrogen.
(2) add 600µl pre-cooled lysate into the mortar, blow it 6 times using 1ml tip, sufficiently mix the tissue powder and the lysate, transfer the mixture into a 1.5ml EP tube, then turn it over 6 times, water bath under 65°C for 20 minutes.
(3) add 3µl RNase, turn over 6 times to mix homogenously, water bath under 37°C for 20 minutes.
(4) cool it to room temperature, add 200µl protein precipitation agent, turn over 6 time to mix homogenously, place it on ice for 5 minutes, 13000 x g centrifuge 4 minute at room temperature.
(5) transfer the supernatant into a new EP tube pre-added with 600µl isopropanol (room temperature), gently mix 6 times, then 13,000 x g centrifuge at room temperate for 1 minutes.
(6) discard the supernatant, add 600µl 70% ethanol (room temperature) into precipitate, 13,000 x g centrifuge at room temperate for 1 minutes.
(7) remove ethanol, air dry for 15 minutes.
(8) add 40µl DNA dissolving solution into the precipitate, incubate at 65°C for 1 hour or 4°C overnight.

### 2. DNA extraction from paraffin embedded tissues

(1) add 1 mg or less tissues into 1.5ml centrifuge tube.
(2) add freshly prepared 100µl incubation buffer/proteinase K solution, and incubate at 56°C overnight based on the type of the samples.
(3) take out the incubated sample tube, add two times volume of lysate buffer.
(4) vortex-oscillate the resin for 10 seconds until the resin is fully suspended, add 7µl fully suspended resin, vortex-oscillate the resin for 3 seconds, then incubate at room temperature for 5 minutes.
(5) vortex-oscillate the resin for 2 seconds, put the tube on a magnetic separation rack (MagneSphere®), immediately conducte magnetic separation.
(6) carefully remove all solution, without touching the resin on the tube wall.
(7) add 100µl lysate buffer, get out the tube from the magnetic separation rack, vortex-oscillate for 2 seconds.
(8) put the tube back to the magnetic separation rack, remove all the lysate.
(9) add 100µl 1x washing fluid, get out the tube from the magnetic separation rack, vortex oscillate 2 seconds.
(10) put the tube back to the magnetic separation rack, remove all the lysate.
(11) repeat step (9) and (10) twice, totally wash three times, and remove all the liquid after the last wash.
(12) open the lid, put the tube on the magnetic separation rack, air dry for 5 minutes.
(13) add 25µl eluate.
(14) close the lid, vortex oscillate for 2 seconds, incubate at 65°C for 5 minutes.
(15) take out the incubated tube, vortex oscillate for 2 seconds, immediately put it on the magnetic separation rack.
(16) carefully transferr the DNA solution into a selected container.

### 3. DNA extraction of whole blood

(1) obtain 300µl anticoagulant whole blood, add 900µl cell lysate, blow 6 times using 1ml tip, so that the whole blood and the cell lysate are sufficiently mixed, place it under room temperature for 10 minutes, blow with the tip three times.
(2) 13,000 x g centrifuge under room temperature for 20 seconds, discard the supernatant, shake violently, add 300µl pre-cooling lysate, blow with 1ml tip until the precipitate are totally dissolved.
(3) add 1.5µl RNase, turn over 6 times to mix homogenously, water bath under 37°C for 20 minutes.
(4) cool to room temperature, add 100µl protein precipitation agent, turn over 6 times to mix homogenously, place it on ice for 5 minutes, 13,000 x g centrifuge under room temperature for 4 minutes.
(5) transfer the supernatant to a new EP tube previously added 300µl isopropanol (room temperature), gently mix 6 times, centrifuge under room temperature for 1 minutes.
(6) discard the supernatant, add 1 ml 70% ethanol (room temperature) into the precipitate, turn over 6 times to mix homogenously, 13,000 x g centrifuge under room temperature for 1 minutes.
(7) remove ethanol, air dry for 15 minutes.
(8) add 40µl DNA dissolving solution, stay at 65°C for 1 hour or 4°C overnight.

### 4. DNA extraction of pleural effusion

(1) obtain 5 ml pleural effusion, 2000 rpm centrifuged at room temperature for 10 minutes, remove the supernatant, add 1 ml cell lysate, turn over 6 times to mix homogenously, stay under room temperature for 10 minutes.
(2) 13,000 x g centrifuged under room temperature for 20 seconds, discard the supernatant, shake violently, add 1 ml pre-cooling lysate, mix until the precipitate totally dissolved.
(3) add 3 µl RNase, turn over 6 times to mix homogenously, water bath under 37°C for 20 minutes.
(4) cool to room temperature, add 200µl protein precipitation agent, turned over 6 times to mix homogenously, place it on ice for 5 minutes, 13,000 x g centrifuged under room temperature for 4 minutes.
(5) transfer the supernatant to a new EP tube previously added 5 ml isopropanol (room temperature), gently mix 6 times, 13,000 x g centrifuged under room temperature for 1 minutes.
(6) discard the supernatant, add 1 ml 70% ethanol (room temperature) into the precipitate, turn over 6 times to mix homogeneously, 13,000 x g centrifuged under room temperature for 1 minutes.
(7) aspirate out ethanol, air dry for 15 minutes.
(8) add 40µl DNA dissolving solution, stay at 65°C for 1 hour or 4°C overnight.

### 5. DNA extraction from cell lines

(1) obtain at least 1 x 10⁶ cells, transfer them into a 1.5 ml EP tube, 13,000 x g centrifuged at room temperature for 10 seconds. If the cells are adherent cells, they should be digested by trypsin before collecting them.
(2) discard the supernatant, add 200 µl PBS to wash the cells, 13,000 x g centrifuged under room temperature for 10 seconds, discard the supernatant, shake violently until the precipitate is suspended.
(3) add 600 µl pre-cooling lysis solution, blow to mix homogenously with 1 ml tip until no visual cell blocks.
(4) add 3 µl RNase, turn over 6 times to mix homogenously, water bath under 37°C for 20 minutes.
(5) cool to room temperature, add 200µl protein precipitation agent, turn over 6 times to mix homogenously, place it on ice for 5 minutes, 13,000 x g centrifuged under room temperature for 4 minutes.
(6) transfer the supernatant to a new EP tube previously added 600µl isopropanol (room temperature), gently mix 6 times, 13,000 x g centrifuged under room temperature for 1 minutes.
(7) discard the supernatant, add 600 µl 70% ethanol (room temperature) into the precipitate, turn over 6 times to mix homogenously, 13,000 x g centrifuged under room temperature for 1 minutes.
(8) aspirate out ethanol, air dry for 15 minutes.
(9) add 40µl DNA dissolving solution, stay at 65°C for 1 hour or 4°C overnight.

### Example 2: Preparation of the plasmid standards containing mutant and wild-type sequences

### 1. Construction of wild-type plasmids (Figure 1, Figure 2)

### 1.1 Preparation of the carrier

TA cloning carrier pMD18-T was purchased from TAKARA Inc.

### 1.2 Preparation of the insert

The insert is prepared using PCR. The template of PCR is the sample genome DNA extracted in Step 1. The reaction system and amplification condition are shown in the following tables (Table 1, Table 2 and Table 3):

**Table 1: PCR reaction system (50µl)**

| reagents | amount(µl/tube) |
|---|---|
| double-distilled water | 29.75 |
| 10x buffer (free of Mg²⁺) | 5 |
| MgCl₂ (25mM) | 7.5 |
| dNTP (10 mM) | 1.25 |
| upstream primer (25µM) | 1.25 |
| downstream primer (25µM) | 1.25 |
| Taq enzyme | 1 |
| DNA template | 3 |
| total volume | 50 |

**Table 2: PCR primers**

| name | Sequence | |
|---|---|---|
| K-ras-F₁ | CCTCTATTGTTGGATCATATT | (SEQ ID NO:3) |
| K-ras-F₂ | AATGACTGAATATAAACTTGTGGTAGT | (SEQ ID NO:4) |
| K-ras-R₁ | TGACTGAATATAAACTTGTGGT | (SEQ ID NO:5) |
| K-ras-R₂ | AAATGATTCTGAATTAGCTGTATCGT | (SEQ ID NO:6) |

**Table 3: PCR amplification condition**

| step | cycles | temperature and time |
|---|---|---|
| step 1 | 1 | 95°C, 1-5 minutes |
| step 2 | 20-30 | 95°C, 10-15 seconds; 55-65°C, 30-60 seconds |

1.3 After recovering the target fragment using QIAgen Gel Recover Kit, insert said fragment into pMD18-T (purchased from TAKARA Inc.) by TA colonizing.
1.4 Amplify the constructed plasmid in E. coli DH5α strain, and harvest by extraction and purification (the methods are showed in Molecular Cloning, A Laboratory Manual, 3rd ed. pages 96-99 and 103.
1.5 Identify the plasmid by double enzyme digestion of BamHI and HindIII.
1.6 Sequence the strains having positive result, and use the strains with correct sequence as the standard containing wild-type sequence (Figure 3).

### 2. Construction of mutant plasmids: design mutant primers of mutant sites, obtain the standards containing mutant sequences by DPN1 method.

2.1 Design the mutant primers (Figure 4) of mutant sites based on the desired mutant sequences.

**Table 4: mutant primers**

| primers name | sequences | |
|---|---|---|
| K-ras-1-F: | AGCTGTTGGCGTAGGCAAGAG | (SEQ ID NO:7) |
| K-ras-1-R: | CGCCAACAGCTCCAACTACCA | (SEQ ID NO:8) |
| K-ras-2-F: | AGCTAGTGGCGTAGGCAAGAG | (SEQ ID NO:9) |
| K-ras-2-R: | CGCCACTAGCTCCAACTACCA | (SEQ ID NO:10) |
| K-ras-3-F: | AGCTGATGGCGTAGGCAAGAG | (SEQ ID NO:11) |
| K-ras-3-R: | CGCCATCAGCTCCAACTACCA | (SEQ ID NO:12) |
| K-ras-4-F: | AGCTTGTGGCGTAGGCAAGAG | (SEQ ID NO:13) |
| K-ras-4-R: | CGCCACAAGCTCCAACTACCA | (SEQ ID NO:14) |
| K-ras-5-F: | CTGGTGACGTAGGCAAGAGTG | (SEQ ID NO:15) |
| K-ras-5-R: | CCTACGTCACCAGCTCCAACT | (SEQ ID NO:16) |

2.2 Use 5ng wild-type plasmid as template, and use mutant primers and Pfu enzyme to mutate the target sites. The amplification system and condition are shown in Table 1, Table 4 and Table 3.
During the preparation of the plasmid containing K-ras Codon 12 GGT → GTT mutant sequence, K-ras-1-F (SEQ ID NO:7) and K-ras-1-R (SEQ ID NO:8) primers are needed to add into the amplification system. During the preparation of the plasmid containing K-ras Codon 12 GGT→AGT mutant sequence, K-ras-2-F (SEQ ID NO:9) and K-ras-2-R (SEQ ID NO:10) primers are needed to add into the amplification system. During the preparation of the plasmid containing K-ras Codon 12 GGT→GAT mutant sequence, K-ras-3-F (SEQ ID NO:11) and K-ras-3-R (SEQ ID NO:12) primers are needed to add into the amplification system. During the preparation of the plasmid containing K-ras Codon 12 GGT→TGT mutant sequence, K-ras-4-F (SEQ ID NO:13) and K-ras-4-R (SEQ ID NO:14) primers are needed to add into the amplification system. During the preparation of the plasmid containing K-ras Codon 13 GGC→GAC mutant sequence, K-ras-5-F (SEQ ID NO:15) and K-ras-5-R (SEQ ID NO:16) primers are needed to add into the amplification system.
2.3 treat the product obtained in step 2.2 with DPN1 enzyme, recover the product after incubating at 37°C for 1 hour, amplify in E. coli DH5α strain, and harvest by extraction and purification.
2.4 Identify the plasmid by double enzyme digestion of BamHI and HindIII.
2.5 Sequence the strains having positive result, and use the strains with correct sequence as the standard containing mutant sequence (Figure 4).

### Example 3: Detection of K-ras mutations from genome DNA of human cell lines, human fresh tumor tissues, peripheral blood, and paraffin embedded tissues, using lung cancer and cervical carcinoma as examples.

1. The templates for fluorescent quantitative PCR are the genome DNA of lung cancer and cervical carcinoma samples extracted in Example 1, and the standards prepared in Example 2. Double-distilled water is served as negative control. For drawing the standard curves, the standards are diluted as 1ng/µl, 0.5ng/µl, 0.25ng/µl, 0.125ng/µl, 0.0625ng/µl, 0.03125ng/µl.
2. The reaction system and condition are shown in Table 2, Table 5, Table 6 and Table 7, wherein the fluorescent emission group bound to the probe is selected from FAM, TET, HEX or ROX, the quench group is selected from BHQ or TAMARA.

**Table 5: Reaction system for fluorescent quantitative PCR (20µl/tube)**

| reagent | amount (µl/tube) |
|---|---|
| double-distilled water | 9.9 |
| 10×buffer (free of Mg²⁺) | 2 |
| MgCl₂ (25mM) | 3 |
| DNTP (10 mM) | 0.5 |
| upstream primer (25µM) | 0.5 |
| downstream primer (25µM) | 0.5 |
| fluorescent probe (25µM) | 0.2 |
| Taq enzyme | 0.4 |
| DNA template | 3 |
| total volume | 20 |

For detecting the mutations in K-ras Codon 12 and 13, it needs to prepare six systems, in which all reagents are same except the probes. Specifically, for detecting K-ras Codon 12 and 13 wild-type genes, it needs to add K-ras-w₁ (SEQ ID NO: 17) or K-ras-w₂ (SEQ ID NO: 18) probes into the system; for detecting K-ras Codon 12 GGT→GTT mutant gene, it needs to add K-ras-1₁ (SEQ ID NO: 19) or K-ras-1₂ (SEQ ID NO: 20) probes into the system; for detecting K-ras Codon 12 GGT→AGT mutant gene, it needs to add K-ras-2₁ (SEQ ID NO: 21) or K-ras-2₂ (SEQ ID NO: 22) probes into the system; for detecting K-ras Codon 12 GGT→GAT mutant gene, it needs to add K-ras-3₁ (SEQ ID NO: 23) or K-ras-3₂ (SEQ ID NO: 24) probes into the system; for detecting K-ras Codon 12 GGT→TGT mutant gene, it needs to add K-ras-4₁ (SEQ ID NO: 25) or K-ras-4₂ (SEQ ID NO: 26) probes into the system; and for detecting K-ras Codon 13 GGC→GAC mutant gene, it needs to add K-ras-5₁ (SEQ ID NO: 27) or K-ras-5₂ (SEQ ID NO: 28) probes into the system.

**Table 6: Probes**

| name | sequence | |
|---|---|---|
| K-ras-w₁ | AGCTGGTGGCGTAGGCAAGA | (SEQ ID NO:17) |
| K-ras-w₂ | AGCTGGTGGCGTAGGCAAGAGT | (SEQ ID NO:18) |
| K-ras-1₁ | AGCT GTT GGCGTAGGCAAGA | (SEQ ID NO:19) |
| K-ras-1₂ | AGCTGTTGGCGTAGGCAAGAGTG | (SEQ ID NO:20) |
| K-ras-2₁ | AGCT AGT GGCGTAGGCAAGA | (SEQ ID NO:21) |
| K-ras-2₂ | AGCTAGTGGCGTAGGCAAGAGTG | (SEQ ID NO:22) |
| K-ras-3₁ | AGCT GAT GGCGTAGGCAAGA | (SEQ ID NO:23) |
| K-ras-3₂ | AGCTGATGGCGTAGGCAAGAGTG | (SEQ ID NO:24) |
| K-ras-4₁ | AGCT TGT GGCGTAGGCAAGA | (SEQ ID NO:25) |
| K-ras-4₂ | AGCTTGTGGCGTAGGCAAGAGTG | (SEQ ID NO:26) |
| K-ras-5₁ | AGCTGGT GAC GTAGGCAAGA | (SEQ ID NO:27) |
| K-ras-5₂ | AGCTGGTGACGTAGGCAAGAGTG | (SEQ ID NO:28) |

**Table 7: Amplification condition**

| steps | Cycles | | temperature and time |
|---|---|---|---|
| step 1 | 1 | | 95°C, 1-5minutes |
| step 2 | 30-45 | 95°C, | 10-15 seconds; 55-65°C (collect fluorescent), 30-60 seconds |

### 3. Drawing the standard curve

The standard curve is drawn based on the CT values obtained from the standard in Step 3. Figure 5 shows the amplification curve of plasmid standard, in which the five rising curves represent, from left to right, the amplification curve of the plasmid standard with the dilute ratio of 0.5ng/µl, 0.25ng/µl, 0.125ng/µl, 0.0625ng/µl and 0.03125ng/µl respectively. The horizontal axis represents cycle number, and the vertical axis represents fluorescent detection value. Accordingly, it is possible to draw the standard curve for calculation (Figure 6). In Figure 6, the horizontal axis represents the logarithm of copy number of the template, the vertical axis represents CT value, wherein the copy number of template=mass/(molecular weight)×6.02×10²³, the molecular weight of plasmid≈the number of bases ×324.5, or is calculated using the software DNAMAN. In the present experiment, the plasmid consists of pMD18-T carrier and an insert. Because the lengths of the insert are almost identical, the biggest difference only lies in twenties bases, which can be ignored with respect to the length of 2692bp for PMD18-T carrier. Therefore, the ratio of copies of wild-type to mutant plasmid standard ≈the ratio of weight.

### 4. Calculation of the ratio of specific K-ras mutation in a sample

According to the standard curve, the copy numbers of wild-type and mutant genome DNA are calculated from the CT values of the sample. Then we obtain the ratio of mutant K-ras DNA to total K-ras DNA (wild-type plus all mutants at said site). As shown in Figure 7, the wild-type CT value of K-ras Codon 12 of a paraffin embedded lung cancer tissue sample is 19.15 (Fig.7A), whereas the value of GGT → TGT mutant is 20.74 (Fig.7B). According to each standard curve formula (Figure 6), we can calculate the copy numbers for them. We then obtain the ratio of the content of mutant to wild-type which was 30:70, and we estimate that about 30% K-ras gene in the tissue sample has GGT→TGT mutation in Codon 12.

### 5. Result of detection

In this Example, we detected the K-ras gene mutation in 48 cases of tissues, whole blood and cell line samples of lung cancer and pancreas cancer, and found that 10 cases had mutations, and the concrete number can be seen in Table 8. The mutation ratios, i.e. the ratio of mutant gene to non-mutant gene in those samples, can be seen in Table 9.

**Table 8: K-ras mutant cases**

| mutation type | case number |
|---|---|
| Codon 12 GGT→GTT | 4 |
| Codon 12 GGT→AGT | 2 |
| Codon 12 GGT→GAT | 1 |
| Codon 12 GGT→TGT | 1 |
| Codon 13 GGC→GAC | 2 |
| total | 10 |

**Table 9: K-ras mutation ratio**

| type of mutant samples | mutation type | mutation ratio |
|---|---|---|
| A549 cell line | Codon 12 GGT→AGT | 30% |
| fresh pancreas cancer tissue | Codon 12 GGT→GTT | 20%, 40%, 40% |
| | Codon 12 GGT→AGT | 25% |
| | Codon 12 GGC→GAC | 20% |
| paraffin embedded lung cancer tissue | Codon 12 GGT→GTT | 22% |
| | Codon 12 GGT→GAT | 35% |
| | Codon 12 GGT→TGT | 28% |
| lung cancer whole blood | Codon 13 GGC→GAC | 35% |

## Claims

1. An assay kit for quantitatively detecting K-ras gene mutation, wherein said mutation is GGT at position 2155 in K-ras Codon 12 being replaced with GTT, AGT, GAT or TGT; or GGC in K-ras Codon 13 being replaced with GAC, comprising:
(1) PCR primers, wherein said primers are SEQ ID NO: 4 and SEQ ID NO: 6;
(2) a probe for fluorescent quantitative PCR, said probe being selected from the group consisting of SEQ ID NO: 19 and SEQ ID NO: 20 for detecting K-ras Codon 12 GGT→GTT mutant gene, SEQ ID NO: 21 and SEQ ID NO: 22 for detecting K-ras Codon 12 GGT→AGT mutant gene, SEQ ID NO: 23 and SEQ ID NO: 24 for detecting K-ras Codon 12 GGT→GAT mutant gene, SEQ ID NO: 25 and SEQ ID NO: 26 for detecting K-ras Codon 12 GGT→TGT mutant gene, SEQ ID NO: 27 and SEQ ID NO: 28 for detecting K-ras Codon 13 GGC→GAC mutant gene, and
(3) a standard comprising both a wild-type plasmid and a mutant plasmid, said wild-type plasmid comprising a wild-type K-ras sequence, and said mutant plasmid comprising a mutant K-ras sequence, said mutant having mutation GGT at position 2155 in K-ras Codon 12 being replaced with GTT, AGT, GAT or TGT; or GGC in K-ras Codon 13 being replaced with GAC.

2. The kit according to claim 1, wherein said probe is linked to a fluorescence emitting group at its 5' end, and is linked to a fluorescence quenching group at its 3' end.

3. The kit according to claim 2, wherein said fluorescence emitting group is FAM, TET, HEX, or ROX; and said fluorescence quenching group is BHQ or TAMARA.

4. The kit according to claim 1, wherein the ratio of said primer to probe is 2:1-10:1, and said primers comprise a forward primer and reverse primer in a ratio of 1:3-3:1.

5. The kit according to claim 1, wherein said wild-type K-ras sequence in said wild-type plasmid is SEQ ID NO:29.

6. The kit according to claim 1, wherein said mutant K-ras sequence in said mutant plasmid is SEQ ID NO:30, SEQ ID NO:31, SEQ ID NO:32, SEQ ID NO:33 or SEQ ID NO:34.

## Patentansprüche

1. Assay-Kit zum quantitativen Nachweis von K-ras Genmutationen, wobei sich die Mutation bei GGT an Position 2155 in K-ras Codon 12 befindet, das durch GTT, AGT, GAT oder TGT ersetzt ist; oder bei GGC in K-ras Codon 13, das durch GAC ersetzt wird, der Folgendes umfasst:
(1) PCR-Primer, wobei die Primer SEQ ID NO: 4 und SEQ ID NO: 6 sind;
(2) eine Sonde für fluoreszierende quantitative PCR, wobei die Sonde ausgewählt ist aus der Gruppe bestehend aus SEQ ID NO: 19 und SEQ ID NO: 20 zum Nachweis der K-ras Codon 12 GGT->GTT-Genmutante, SEQ ID NO: 21 und SEQ ID NO: 22 zum Nachweis der K-ras Codon 12 GGT->AGT Genmutante, SEQ ID NO: 23 und SEQ ID NO: 24 zum Nachweis der K-ras Codon 12 GGT->GAT Genmutante, SEQ ID NO: 25 und SEQ ID NO: 26 zum Nachweis der K-ras Codon 12 GGT->TGT Genmutante, SEQ ID NO: 27 und SEQ ID NO: 28 zum Nachweis der K-ras Codon 13 GGC->GAC Genmutante, und
(3) einen Standard, der sowohl ein Wildtyp-Plasmid als auch ein mutiertes Plasmid umfasst, wobei das Wildtyp-Plasmid eine Wildtyp-K-ras Sequenz umfasst und das mutierte Plasmid eine mutierte K-ras Sequenz umfasst, wobei die Mutante die Mutation aufweist, dass GGT an Position 2155 in K-ras Codon 12 durch GTT, AGT, GAT oder TGT ersetzt ist; oder GGC in K-ras Codon 13 durch GAC ersetzt ist.

2. Kit nach Anspruch 1, wobei die Sonde an ihrem 5'-Ende mit einer Fluoreszenz-emittierenden Gruppe verbunden ist und an ihrem 3'-Ende mit einer Fluoreszenz-Quench Gruppe verbunden ist.

3. Kit nach Anspruch 2, wobei die Fluoreszenzemittierende Gruppe FAM, TET, HEX oder ROX ist; und die Fluoreszenz-Quench Gruppe BHQ oder TAMARA ist.

4. Kit nach Anspruch 1, wobei das Verhältnis des Primers zur Sonde 2:1-10:1 beträgt und die Primer einen Vorwärts-Primer und einen Rückwärts-Primer im Verhältnis von 1:3-3:1 umfassen.

5. Kit nach Anspruch 1, wobei die Wildtyp-K-ras Sequenz in dem Wildtyp-Plasmid SEQ ID NO: 29 ist.

6. Kit nach Anspruch 1, wobei die mutierte K-ras-Sequenz in dem mutierten Plasmid SEQ ID NO: 30, SEQ ID NO: 31, SEQ ID NO: 32, SEQ ID NO: 33 oder SEQ ID NO: 34 ist.

## Revendications

1. Trousse d'essai pour détection quantitative de mutations du gène K-ras, lesquelles mutations sont le remplacement de GGT dans le Codon 12 de K-ras, en position 2155, par GTT, AGT, GAT ou TGT, ou le remplacement de GGC dans le Codon 13 de K-ras par GAC, laquelle trousse comprend :
1) des amorces pour PCR, lesquelles amorces sont la Séquence N° 4 et la Séquence N° 6,
2) une sonde pour PCR quantitative par fluorescence, laquelle sonde est choisie dans l'ensemble formé par les suivantes :
Séquence N° 19 et Séquence N° 20, pour détecter le gène portant, dans le Codon 12 de K-ras, la mutation GGT→GTT,
Séquence N° 21 et Séquence N° 22, pour détecter le gène portant, dans le Codon 12 de K-ras, la mutation GGT→AGT,
Séquence N° 23 et Séquence N° 24, pour détecter le gène portant, dans le Codon 12 de K-ras, la mutation GGT→GAT,
Séquence N° 25 et Séquence N° 26, pour détecter le gène portant, dans le Codon 12 de K-ras, la mutation GGT→TGT,
Séquence N° 27 et Séquence N° 28, pour détecter le gène portant, dans le Codon 13 de K-ras, la mutation GGC→GAC,
3) et un étalon comprenant à la fois un plasmide de type sauvage et un plasmide mutant, lequel plasmide de type sauvage comprend une séquence K-ras de type sauvage et lequel plasmide mutant comprend une séquence K-ras mutante, laquelle séquence mutante porte une mutation qui est le remplacement de GGT dans le Codon 12 de K-ras, en position 2155, par GTT, AGT, GAT ou TGT, ou le remplacement de GGC dans le Codon 13 de K-ras par GAC.

2. Trousse conforme à la revendication 1, dans laquelle ladite sonde est raccordée, au niveau de son extrémité 5', à un groupe émetteur de fluorescence, et au niveau de son extrémité 3', à un groupe extincteur de fluorescence.

3. Trousse conforme à la revendication 2, dans laquelle ledit groupe émetteur de fluorescence est FAM, TET, HEX ou ROX, et ledit groupe extincteur de fluorescence est BHQ ou TAMARA.

4. Trousse conforme à la revendication 1, dans laquelle le rapport desdites amorces à la sonde vaut de 2/1 à 10/1, et lesdites amorces comprennent une amorce sens et une amorce anti-sens en un rapport valant de 1/3 à 3/1.

5. Trousse conforme à la revendication 1, dans laquelle ladite séquence K-ras de type sauvage présente dans ledit plasmide de type sauvage est la Séquence N° 29.

6. Trousse conforme à la revendication 1, dans laquelle ladite séquence K-ras mutante présente dans ledit plasmide mutant est la Séquence N° 30, la Séquence N° 31, la Séquence N° 32, la Séquence N° 33 ou la Séquence N° 34.


### SEQUENCE LISTING

<110> Beijing ACCB Biotech Ltd.
<120> AN ASSAY KIT FOR QUANTITATIVELY DETECTING K-RAS GENE MUTATIONS
<130> ykb007p
<160> 34
<170> PatentIn version 3.3
<210> 1
   <211> 290
   <212> DNA
   <213> Unknown
<220>
   <223> Sequences of K-ras Exon 1 and 2
<300>
   <308> NCBI NM_004985, NM_033360
<400> 1
   atgactgaat ataaacttgt ggtagttgga gctggtggcg taggcaagag tgccttgacg     60
   atacagctaa ttcagaatca ttttgtggac gaatatgatc caacaataga ggattcctac    120
   aggaagcaag tagtaattga tggagaaacc tgtctcttgg atattctcga cacagcaggt    180
   caagaggagt acagtgcaat gagggaccag tacatgagga ctggggaggg ctttctttgt    240
   gtatttgcca taaataatac taaatcattt gaagatattc accattatag               290
<210> 2
   <211> 425
   <212> DNA
   <213> Unknown
<220>
   <223> a part of wild-type DNA sequence of K-ras
<300>
   <308> NCBI NT_000012.11
<400> 2
   caactggaat tttcatgatt gaattttgta aggtattttg aaataatttt tcatataaag      60
   gtgagtttgt attaaaaggt actggtggag tatttgatag tgtattaacc ttatgtgtga     120
   catgttctaa tatagtcaca ttttcattat ttttattata aggcctgctg aaaatgactg     180
   aatataaact tgtggtagtt ggagctggtg gcgtaggcaa gagtgccttg acgatacagc     240
   taattcagaa tcattttgtg gacgaatatg atccaacaat agaggtaaat cttgttttaa     300
   tatgcatatt actggtgcag gaccattctt tgatacagat aaaggtttct ctgaccattt     360
   tcatgagtac ttattacaag ataattatgc tgaaagttaa gttatctgaa atgtaccttg     420
   ggttt                                                                 425
<210> 3
   <211> 21
   <212> DNA
   <213> Artificial
<220>
   <223> upstream primer of K-ras
<400> 3
   cctctattgt tggatcatat t 21
<210> 4
   <211> 27
   <212> DNA
   <213> Artificial
<220>
   <223> upstream primer of K-ras
<400> 4
   aatgactgaa tataaacttg tggtagt 27
<210> 5
   <211> 22
   <212> DNA
   <213> Artificial
<220>
   <223> downstream primer of K-ras
<400> 5
   tgactgaata taaacttgtg gt 22
<210> 6
   <211> 26
   <212> DNA
   <213> Artificial
<220>
   <223> downstream primer of K-ras
<400> 6
   aaatgattct gaattagctg tatcgt 26
<210> 7
   <211> 21
   <212> DNA
   <213> Artificial
<220>
   <223> upstream primer of K-ras Codon 12 GGT->GTT mutation
<400> 7
   agctgttggc gtaggcaaga g 21
<210> 8
   <211> 21
   <212> DNA
   <213> Artificial
<220>
   <223> downstream primer of K-ras Codon 12 GGT->GTT mutation
<400> 8
   cgccaacagc tccaactacc a 21
<210> 9
   <211> 21
   <212> DNA
   <213> Artificial
<220>
   <223> upstream primer of K-ras Codon 12 GGT -> AGT mutation
<400> 9
   agctagtggc gtaggcaaga g 21
<210> 10
   <211> 21
   <212> DNA
   <213> Artificial
<220>
   <223> downstream primer of K-ras Codon 12 GGT -> AGT mutation
<400> 10
   cgccactagc tccaactacc a 21
<210> 11
   <211> 21
   <212> DNA
   <213> Artificial
<220>
   <223> upstream primer of K-ras Codon 12 GGT -> GAT mutation
<400> 11
   agctgatggc gtaggcaaga g 21
<210> 12
   <211> 21
   <212> DNA
   <213> Artificial
<220>
   <223> downstream primer of K-ras Codon 12 GGT -> GAT mutation
<400> 12
   cgccatcagc tccaactacc a 21
<210> 13
   <211> 21
   <212> DNA
   <213> Artificial
<220>
   <223> upstream primer of K-ras Codon 12 GGT -> TGT mutation
<400> 13
   agcttgtggc gtaggcaaga g 21
<210> 14
   <211> 21
   <212> DNA
   <213> Artificial
<220>
   <223> downstream primer of K-ras Codon 12 GGT -> TGT mutation
<400> 14
   cgccacaagc tccaactacc a 21
<210> 15
   <211> 21
   <212> DNA
   <213> Artificial
<220>
   <223> upstream primer of K-ras Codon 13 GGC -> GAC mutation
<400> 15
   ctggtgacgt aggcaagagt g 21
<210> 16
   <211> 21
   <212> DNA
   <213> Artificial
<220>
   <223> downstream primer of K-ras Codon 13 GGC -> GAC mutation
<400> 16
   cctacgtcac cagctccaac t 21
<210> 17
   <211> 20
   <212> DNA
   <213> Artificial
<220>
   <223> wild-type probe of K-ras
<400> 17
   agctggtggc gtaggcaaga 20
<210> 18
   <211> 22
   <212> DNA
   <213> Artificial
<220>
   <223> wild-type probe of K-ras
<400> 18
   agctggtggc gtaggcaaga gt 22
<210> 19
   <211> 20
   <212> DNA
   <213> Artificial
<220>
   <223> mutant probe of K-ras Codon 12 GGT -> GTT mutation
<400> 19
   agctgttggc gtaggcaaga 20
<210> 20
   <211> 23
   <212> DNA
   <213> Artificial
<220>
   <223> mutant probe of K-ras Codon 12 GGT -> GTT mutation
<400> 20
   agctgttggc gtaggcaaga gtg 23
<210> 21
   <211> 20
   <212> DNA
   <213> Artificial
<220>
   <223> mutant probe of K-ras Codon 12 GGT -> AGT mutation
<400> 21
   agctagtggc gtaggcaaga 20
<210> 22
   <211> 23
   <212> DNA
   <213> Artificial
<220>
   <223> mutant probe of K-ras Codon 12 GGT -> AGT mutation
<400> 22
   agctagtggc gtaggcaaga gtg 23
<210> 23
   <211> 20
   <212> DNA
   <213> Artificial
<220>
   <223> mutant probe of K-ras Codon 12 GGT -> GAT mutation
<400> 23
   agctgatggc gtaggcaaga 20
<210> 24
   <211> 23
   <212> DNA
   <213> Artificial
<220>
   <223> mutant probe of K-ras Codon 12 GGT -> GAT mutation
<400> 24
   agctgatggc gtaggcaaga gtg 23
<210> 25
   <211> 20
   <212> DNA
   <213> Artificial
<220>
   <223> mutant probe of K-ras Codon 12 GGT -> TGT mutation
<400> 25
   agcttgtggc gtaggcaaga 20
<210> 26
   <211> 23
   <212> DNA
   <213> Artificial
<220>
   <223> mutant probe of K-ras Codon 12 GGT -> TGT mutation
<400> 26
   agcttgtggc gtaggcaaga gtg 23
<210> 27
   <211> 20
   <212> DNA
   <213> Artificial
<220>
   <223> mutant probe of K-ras Codon 13 GGC -> GAC mutation
<400> 27
   agctggtgac gtaggcaaga 20
<210> 28
   <211> 23
   <212> DNA
   <213> Artificial
<220>
   <223> mutant probe of K-ras Codon 13 GGC -> GAC mutation
<400> 28
   agctggtgac gtaggcaaga gtg 23
<210> 29
   <211> 41
   <212> DNA
   <213> Unknown
<220>
   <223> DNA sequence containing wild-type sequence of K-ras Codon 12 and 13
<400> 29
   cttgtggtag ttggagctgg tggcgtaggc aagagtgcct t 41
<210> 30
   <211> 41
   <212> DNA
   <213> Unknown
<220>
   <223> DNA sequence containing K-ras Codon 12 GTT mutant sequence
<400> 30
   cttgtggtag ttggagctgt tggcgtaggc aagagtgcct t 41
<210> 31
   <211> 40
   <212> DNA
   <213> Unknown
<220>
   <223> DNA sequence containing K-ras Codon 12 AGT mutant sequence
<400> 31
   ttgtggtagt tggagctagt ggcgtaggca agagtgcctt 40
<210> 32
   <211> 40
   <212> DNA
   <213> Unknown
<220>
   <223> DNA sequence containing K-ras Codon 12 GAT mutant sequence
<400> 32
   ttgtggtagt tggagctgat ggcgtaggca agagtgcctt 40
<210> 33
   <211> 41
   <212> DNA
   <213> Unknown
<220>
   <223> DNA sequence containing K-ras Codon 12 TGT mutant sequence
<400> 33
   cttgtggtag ttggagcttg tggcgtaggc aagagtgcct t 41
<210> 34
   <211> 40
   <212> DNA
   <213> Unknown
<220>
   <223> DNA sequence containing K-ras Codon 13 GAC mutant sequence
<400> 34
   ttgtggtagt tggagctggt gacgtaggca agagtgcctt 40